Europäisches Patentamt

European Patent Office

Office européen des brevets

(11) Publication number: **0 081 980**
**B1**

# EUROPEAN PATENT SPECIFICATION

(45) Date of publication of patent specification: **26.02.86**

(51) Int. Cl.⁴: **F 23 M 11/04**

(21) Application number: **82306582.6**

(22) Date of filing: **09.12.82**

(54) Systems for determining the efficiency of fossil fuel-fired vapour generators.

(30) Priority: **10.12.81 US 329538**

(43) Date of publication of application:
**22.06.83 Bulletin 83/25**

(45) Publication of the grant of the patent:
**26.02.86 Bulletin 86/09**

(84) Designated Contracting States:
**DE FR GB IT**

(56) References cited:
**GB-A- 510 525**
**GB-A-2 016 707**
**US-A-1 591 444**
**US-A-2 723 559**

(73) Proprietor: **THE BABCOCK & WILCOX COMPANY**
**1010 Common Street P.O. Box 60035**
**New Orleans Louisiana 70160 (US)**

(72) Inventor: **Johnson, Ralph K.**
**652 Lander Drive**
**Highland Heights Ohio 44143 (US)**
Inventor: **Keyes, Marion A.**
**120 Riverstone Drive**
**Chagrin Falls Ohio 44022 (US)**
Inventor: **Kaya, Azmi**
**2365 Woodpark Road**
**Akron Ohio 44313 (US)**
Inventor: **Moss, William H.**
**34996 N. Turtle Trail**
**Willoughby Ohio 44094 (US)**

(74) Representative: **Cotter, Ivan John et al**
**D. YOUNG & CO. 10 Staple Inn**
**London WC1V 7RD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Description

This invention relates to systems for determining the efficiency of fossil fuel-fired vapour generators.

One technique for determining the efficiency of a fossil fuel-fired steam generator, as set forth in the ASME/ANSI Power Test Codes, is the so-called Heat Loss Method which is based on the calculation of heat losses per unit weight (e.g. pound) of fuel. The generator efficiency is then the difference between the higher heating value of the fuel minus the total of the calculated heat losses, expressed in percent, for given feedwater conditions.

A complete efficiency test of a fossil fuel-fired steam generator as presently conducted requires many man-hours of labour to record the required data at stipulated increments of time over an extended period of time and then to make the necessary calculations at each of several loads. Furthermore, to obtain meaningful results during the period of time in which the tests are conducted, the generator must be held in a steady-state condition. For these reasons efficiency tests are usually conducted only when required to meet performance guarantees.

Published UK Patent Application No. GB—A—2 016 707 discloses a flue gas loss or combustion efficiency determining apparatus primarily for use in furnaces using natural gas as a fuel. It is acknowledged that the apparatus ignores some of the heat loss factors contributing to total heat loss. The apparatus evaluates loss by means of an algorithm in the form of an equation including several terms. There is no indication whether individual terms of the equation relate to individual heat loss factors. In particular, there is no indication that the apparatus derives a signal proportional to the heat loss in the dry fuel gas which, as known to those skilled in the art, represents a significant proportion of the total loss.

The present invention seeks to provide a system by which the efficiency of a fossil fuel-fired vapour generator may be automatically and continuously monitored and in which a signal proportional to the heat loss in the dry flue gas is developed.

According to the present invention there is provided a system for the automatic and continuous determination of the efficiency of a fossil fuel-fired vapour generator, the system being operative to generate a plurality of heat loss signals proportional to a plurality of different heat losses and to sum the heat loss signals to produce a signal proportional to the vapour generator efficiency, and the system including means for generating a first signal proportional to the weight units of dry exit flue gas per unit weight of fuel, means for generating a second signal corresponding to the temperature difference between the exit flue gas and air supplied for combustion, means for generating a third signal proportional to the specific heat of the dry exit flue gases, and means for generating a fourth signal proportional to the product of the first, second and third signals and to the heat loss in the dry flue gas in thermal units per unit weight of fuel.

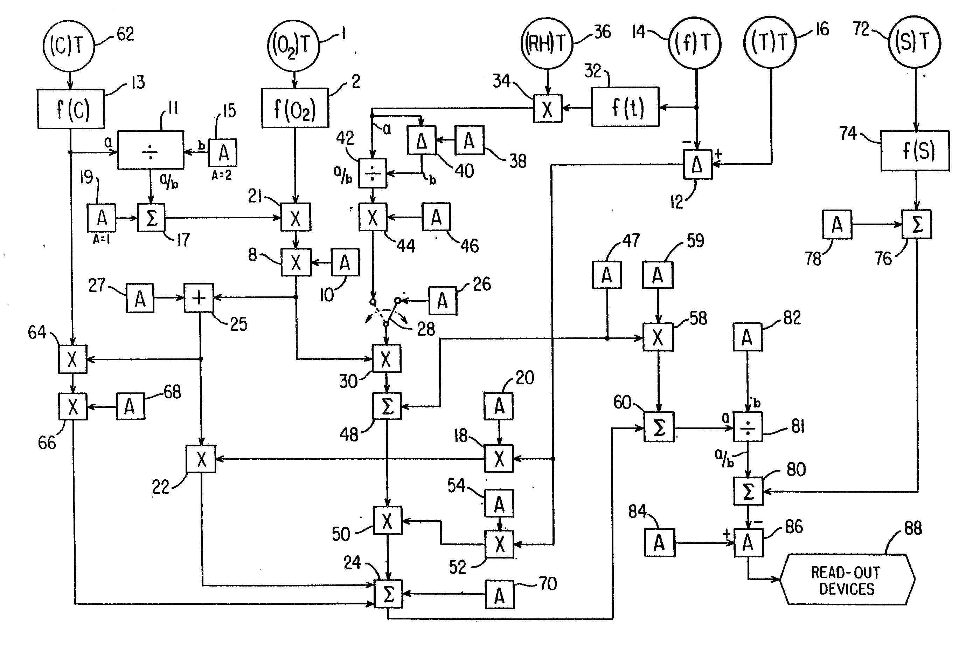

The invention will now be further described, by way of illustrative and non-limiting example, with reference to the accompanying drawing, the sole figure of which is a logic diagram of a system embodying this invention.

In the drawing, conventional logic symbols have been used. It will be recognised that the components, or hardware, as it is sometimes called, which such symbols represent are commercially available and their operation well understood by those familiar with the art. Further, conventional logic symbols have been used to avoid specific identification of this invention with any particular type of components such as analog or digital, as this invention comprehends either one or a combination of such types.

Referring now to the drawing, excess air in flue gas of a fossil fuel-fired vapour generator is determined by means of an oxygen transducer 1 generating a signal transmitted to a function generator 2, an output signal from which is proportional to the total air supplied for combustion in percent by weight.

There is a substantially constant relationship between oxygen content in the flue gas and total air regardless of the fuel being burned, particularly as in modern steam generators the excess air can be maintained at low values in the region of 20 percent or less. If required, however, the output signal from the function generator 2 may be adjusted to compensate for changes in the proportionality between oxygen content and total air occasioned by a change in fuel.

A signal proportional to the weight units (kg) of air supplied for combustion per unit weight (kg) of fuel is obtained by multiplying, in a multiplying unit 8, the signal from the function generator 2, by a signal proportional to the theoretical weight (kg) of air required per unit weight (kg) of fuel, generated in a manually adjustable unit 10.

Oxygen analysers of the so-called electrochemical type using a Zirconium-oxide sensor operate at approximately 815°C. At this temperature unburned combustibles, such as CO and $H_2$, react with the oxygen present in the flue gas. As a result, the signal generated by the oxygen transducer 1 is proportional to the excess oxygen remaining in the flue gas based on complete combustion. If such an oxygen transmitter is incorporated in the system, a correction to the output signal from the function generator 2 is made by dividing in half, by means of a divider unit 11 and a signal generator 15, the output signal from a function generator 13 responsive to the signal generated in a combustibles transducer 62. The output signal from the divider unit 11 is then summed in a summing unit 17 with a constant value signal generated in a signal generator 19. The output signal from the function generator 2 is then multiplied in a multiplier unit 21 producing an output signal proportional to the weight (in kg) of dry air in weight units (kg) per unit weight (kg) of fuel. If the oxygen transducer is of a type having an operating temperature below the temperature at which the unburned combustibles react with the oxygen present in the flue gas, such as the so-called catalytic combustion or paramagnetic types, then no such correction is required.

The weight (kg) of dry exit flue gas per unit weight (kg) of fuel is obtained by adding, in an adder unit 25, to the output signal from multiplier unit 8, a signal proportional to the weight of combustibles in a weight unit (kg) of fuel generated in a signal generator 27.

Having thus obtained the weight (kg) of dry air and weight (kg) of dry flue gas per unit weight (kg) of fuel, the individual losses due to heat in the exit flue gas, moisture in the air supplied for combustion, sensible and latent heat in the $H_2O$ in the fuel and combustibles in the flue gas (if any) are determined as follows:

Heat loss in dry flue gas (J/kg of fuel)　　(1)

$$L_1 = kg\ dry\ flue\ gas/kg\ of\ fuel \times 1034 \times (T-t)$$

where:
$L_1$=Heat loss in dry flue gas in J/kg fuel
T=Flue gas temperature (K)
t=Ambient temperature (K)
1034=Specific heat of dry air in J/kg · K at 101 kPa absolute.

It has been assumed that the specific heat of the dry flue mass is very nearly equal to the specific heat of dry air.

A signal proportional to the difference in ambient and exit flue gas temperatures is generated in a difference unit 12 responsive to the output signal from an ambient temperature transducer 14 and a flue gas exit temperature transducer 16. The signal generated in the difference unit 12 is then multiplied in a multiplying unit 18 by the output signal from a signal generator 20 proportional to the specific heat of the dry flue gas. To obtain a signal proportional to the heat loss in the exit dry flue gas per unit weight (pound) of fuel the output signal from the multiplying unit 18 is multiplied in a multiplying unit 22 by the output signal from the adder unit 25. The output signal from the multiplying unit 22 forms one input to a loss summing unit 24.

Sensible heat loss due to moisture in the combustion air and fuel　　　　(2)

$$L_2 = (kg\ dry\ air/kg\ fuel \times (y) + kg\ moist.\ per\ kg\\ of\ fuel) \times specific\ heat\ of\ H_2O \times (T-t)$$

where:
$L_2$=Sensible heat loss due to moisture in air and fuel
y=Weight (kg) of moisture in combustion air per kg of combustion air
Conveniently, $L_2$ can be determined by first determining the total moisture in the combustion air and fuel, then determining the heat loss per unit weight (kg) of moisture, the product of the two determinations being the sensible heat loss $L_2$.

The amount of moisture in the combustion air is often taken as y=0.013 weight units (kg) per unit weight (kg) of dry air, corresponding to conditions of 27°C ambient temperature and 60 percent relative humidity; or y can be calculated from measurements of ambient air temperature and relative humidity for varying weather conditions.

When the moisture content y is assumed to be 0.013 weight units (kg) per unit weight (kg) of dry air, a signal proportional thereto is generated in a signal generator 26 and transmitted through a selector switch 28 to a multiplying unit 30 for multiplication by the output signal from the multiplier unit 8 to produce a signal proportional to the weight units (kg) of moisture in the weight units (kg) of combustion air per unit weight (kg) of fuel.

When moisture is calculated from measurements of ambient air temperature and relative humidity, a signal proportional to the vapour pressure of $H_2O$ at ambient temperature is first generated by means of a function generator 32 responsive to the signal generated in the ambient temperature transducer 14, which is multiplied in a multiplier unit 34 by a signal proportional to the relative humidity of the ambient air supplied for combustion, generated in a relative humidity transducer 36. The output signal from the multiplier unit 34 is then modified in accordance with changes in atmospheric pressure by means of a signal generator 38 generating a signal corresponding to ambient pressure which is subtracted from the output signal (a) from the multiplying unit 34 in a difference unit 40, producing an output signal (b) by which the output signal (a) is divided in a divider unit 42. The output signal from the divider unit 42 is then multiplied, in a multiplier unit 44, by a signal corresponding to the ratio between the molecular weight of water vapour and air, generated in a signal generator 46, to produce a signal proportional to the weight units (kg) of water vapour per unit weight (kg) of air supplied for combustion. This signal may then be transmitted through the selector switch 28 to the multiplier unit 30 to produce the signal proportional to the weight units (kg) of moisture in the weight units (kg) of combustion air per unit weight (kg) of fuel.

A signal proportional to the weight units (kg) of moisture in a unit weight (kg) of fuel is generated in a signal generator 47 and transmitted to a summing unit 48 wherein it is added to the signal from the multiplying unit 30 to produce an output signal proportional to the total moisture in the combustion air and fuel per unit weight (kg) of fuel. This output signal is then multiplied in a multiplier unit 50 by a signal proportional to the heat in thermal units (J) wasted in the flue gases per unit weight (kg) of moisture to produce a signal proportional to the total loss per unit weight (kg) of fuel which is transmitted to the loss summing unit 24. The heat wasted in the flue gases per unit weight (pound) of moisture is obtained by multiplying the signal from difference unit 12 in a multiplier unit 52 by a signal proportional to the specific heat of water vapour, usually taken as 1989 J/kg · K, generated in a signal generator 54.

Heat loss due to vapourisation of water in the fuel (3)

$$L_3 = \text{kg water in fuel/kg of fuel} \times 2.440 \times 10^6$$

where:

$L_3$=Heat loss due to vapourisation of water in the fuel

$2.440 \times 10^6$=latent heat of vapourisation in J/kg of water

The signal proportional to the moisture in the fuel, generated in the signal generator 47, is multiplied, in a multiplier unit 58, by a signal proportional to the heat of vapourisation (assumed to be $2.440 \times 10^6$ J/kg of water) generated in a second generator 59. The output signal from the multiplier unit 58 is transmitted to a loss summing unit 60.

Heat loss due to unburned combustibles in the flue gas (4)

$$L_4 = \% \text{ combustibles in the flue gas} \times \text{kg dry products of combustion/(kg fuel} \times 22.67 \times 10^6)$$

where:

$L_4$=Loss due to unburned combustibles

$22.67 \times 10^6$=Heat of combustion in J/kg of CO.

While by good design and careful operation no unburned gaseous combustibles will be found in the flue gases, under certain conditions they may be unavoidable. The usual gaseous combustible is CO which, if present, materially reduces steam generator efficiency. The loss in thermal units (J) per unit weight (kg) of fuel is determined by means of the transducer 62 and the function generator 13 generating a signal proportional to the percent combustibles present in the flue gas which is multiplied, in a multiplier unit 64, by the output signal from the adder unit 25 to obtain a signal proportional to the weight units (kg) of CO per unit weight (kg) of fuel. The output signal from the multiplier 64 is then multiplied, in a multiplier unit 66, by the output signal from a signal generator 68 proportional to the heat of combustion of CO, which is $22.67 \times 10^6$ per kg of CO. The signal from a multiplier unit 66, proportional to the BTU loss per pound of fuel, forms one input signal to the loss summing unit 24.

Heat loss due to unburned carbon in the ash (5)

$$L_5 = \text{kg ash/kg fuel} \times \frac{\text{kg carbon/kg ash}}{1 - \text{kg carbon/kg ash}} \times 33.73 \times 10^6$$

where:

$L_5$=Loss in J/kg of fuel due to carbon in the ash

$33.73 \times 10^6$=heat of combustion J/kg, carbon to carbon dioxide

This calculation requires an analysis of the ash to determine the weight units (kg) of carbon in the ash per unit weight (kg) of ash. The loss in J per kg of fuel is then determined by means of equation (5). As shown in the drawing, a signal proportional to the loss is generated in a signal generator 70 which is transmitted to the loss summing unit 24.

Heat loss due to heat radiation (6)

$$L_6 = \text{A Constant} \times (\text{Fractional Load})^{-0.95}$$

where:

$L_6$=Radiation loss in percent of heat input

The heat loss due to radiation varies inversely in non-linear functional relationship to generator load as expression in equation (6). As shown in the drawing, a signal proportional to the fractional boiler load is generated in a steam flow transducer 72 and a signal proportional to the non-linear functional relationship between fractional load and radiation loss is generated in a function generator 74.

Unaccounted for losses

In addition to the loss due to heat radiation, there are certain other losses due, for example, to heat leaks and blow down. These losses are based on experiences reported in the literature and are expressed as percent losses and accordingly may be added to the signal output of the function generator 74 by means of a summing unit 76. A signal generated in a signal generator 78 is connected to an input of the summing unit 76 to produce an output signal proportional to the total of radiation and unaccounted for losses in percent which is transmitted to a summing unit 80.

Determination of vapour generator efficiency

The losses summed in the summing unit 60, expressed in thermal units (J) per unit weight (kg) of fuel, are converted to losses in percent by dividing the output signal from the summing unit 60, in a divider unit 81, by a signal proportional to the higher heating value of the fuel generated in a signal generator 82, to produce a signal proportional to the losses summed in the summing unit 60 expressed in percent. The output signal from the divider unit 81 is then summed in the summing unit 80 with the signal from the summing unit 76 to obtain an output signal proportional to the total percent losses, which is converted to a signal proportional to boiler efficiency by subtracting the output signal, in a difference unit 86, from a signal having a constant value of 100, generated in a signal generator 84. The output signal from the difference unit 86 is transmitted to such read-out devices, schematically illustrated at 88, as are required to meet the exigencies of a particular application.

## Claims

1. A system for the automatic and continuous determination of the efficiency of a fossil fuel-fired vapour generator, the system being operative to generate a plurality of heat loss signals proportional to a plurality of different heat losses and to sum the heat loss signals to produce a signal proportional to the vapour generator efficiency, and the system including means (25) for generating a first signal proportional to the weight units of dry exit flue gas per unit weight of fuel, means (12, 14, 16) for generating a second signal corresponding to the temperature difference between the exit flue gas and air supplied for combustion, means (20) for generating a third signal proportional to the specific heat of the dry exit flue gases, and means (18, 22) for generating a fourth signal proportional to the product of the first, second and third signals and to the heat loss in the dry flue gas in thermal units per unit weight of fuel.

2. A system according to claim 1, including means (62, 64) for generating a fifth signal proportional to the weight units of combustibles in the exit flue gas per unit weight of fuel, means (68) for generating a sixth signal proportional to the heat of combustion of the combustibles, and means (66) for generating a seventh signal proportional to the product of the fifth and sixth signals and to the heat loss due to combustibles in the exit flue gas in thermal units per unit weight of fuel.

3. A system according to claim 2, including means (26, 30) for generating an eighth signal proportional to the weight units of moisture in the air supplied for combustion per unit weight of fuel, means (47) for generating a ninth signal proportional to the weight units of moisture in the fuel per unit weight of fuel, means (48) for generating a tenth signal proportional to the sum of the eighth and ninth signals, mèans (54) for generating an eleventh signal proportional to the specific heat of water vapour, means (52) for generating a twelfth signal proportional to the product of the second and eleventh signals, and means (50) for generating a thirteenth signal proportional to the product of the tenth and twelfth signals and to the heat loss due to moisture in the fuel and air supplied for combustion per unit weight of fuel.

4. A system according to claim 3, including means (59) for generating a fourteenth signal proportional to the latent heat of vapourisation of water in the fuel, and means (58) for generating a fifteenth signal proportional to the product of the ninth and fourteenth signals and to the loss due to the latent heat of vapourisation of the water in the fuel.

5. A system according to claim 4, including means (72) for generating a sixteenth signal proportional to the rate of vapour flow from the generator, and a function generator (74) responsive to the sixteenth signal for generating a seventeenth signal varying in inverse non-linear relationship to the magnitude of the sixteenth signal and to the heat loss in percent due to heat radiation from the vapour generator.

6. A system according to claim 5, pncluding means (70) for generating an eighteenth signal proportional to the heat loss due to the unburned carbon in ash resulting from combustion of the 'fuel.

7. A system according to claim 6, including means (24, 60) for generating a nineteenth signal proportional to the sum of the fourth, seventh, thirteenth, fifteenth and eighteenth signals, means (82) for generating a twentieth signal proportion, to the higher heating value of fuel, means (81) for generating a twenty-first signal proportional to the nineteenth signal divided by the twentieth signal, means (80) for generating a twenty-second signal proportional to the sum of the seventeenth and twenth-first signals, and to the total of the said heat losses in percent, means (84) for generating a twenty-third signal proportional to 100% efficiency of the vapour generator and, means (86) for generating a twenty-fourth signal which is proportional to the difference between the twenty-third and twenty-second signals and constitutes said signal proportional to the vapour generator efficiency.

## Patentansprüche

1. Anlage für die automatische und kontinuierliche Ermittlung des Wirkungsgrades eines mit fossilen Brennstoffen beheizten Dampfgenerators, wobei die Anlage betrieblich so ausgestaltet ist, daß eine Vielzahl von Wärmeverlustsignalen erzeugt wird, die proportional einer Vielzahl von unterschiedlichen Wärmeverlusten ist, zur Summation der Wärmeverlustsignale zur Erzeugung eines Signals, welches proportional dem Wirkungsgrad des Dampfgenerators ist, und wobei die Anlage Mittel (25) aufweist für die Erzeugung eines ersten Signals, welches proportional zu den Gewichtseinheiten von trockenem Ausgangsrauchgas pro Einheitsgewicht Brennstoff ist, sowie Mittel (12, 14, 16) aufweist für die Erzeugung eines zweiten Signals, welches der Temperaturdifferenz entspricht zwischen dem Ausgangsrauchgas und für die Verbrennung zugeführter Luft, Mittel (20) aufweist für die Erzeugung eines dritten Signals, welches der spezifischen Wärme der trockenen Ausgangsrauchgase proportional ist, und Mittel (18, 22) aufweist für die Erzeugung eines vierten Signals, welches dem Produkt des ersten, zweiten und dritten Signals und dem Wärmeverlust in dem trockenen Rauchgas in Wärmeeinheiten pro Einheitsgewicht Brennstoff proportional ist.

2. Anlage nach Anspruch 1 mit Mitteln (62, 64) für die Erzeugung eines fünften Signals, welches den Gewichtseinheiten von Brennstoffen im Ausgangsrauchgas pro Einheitsgewicht Brennstoff proportional ist, Mitteln (68) zur Erzeugung eines sechsten Signals, welches der Wärme der Verbrennung der Brennstoffe proportional ist, und Mitteln (66) für die Erzeugung eines siebten Signals, welches dem Produkt der fünften und sechsten Signale und dem Wärmeverlust infolge Brennstoffen im Ausgangsrauchgas in Wärmeeinheiten pro Einheitsgewicht Brennstoff proportional ist.

3. Anlage nach Anspruch 2 mit Mitteln (26, 30) für die Erzeugung eines achten Signals, welches den Gewichtseinheiten der Feuchtigkeit in der Luft proportional ist, die für die Verbrennung pro Einheitsgewicht Brennstoff zugeführt wird, Mitteln (47) für die Erzeugung eines neunten Signals, welches den Gewichtseinheiten der Feuchtigkeit im Brennstoff pro Einheitsgewicht Brennstoff proportional ist, Mitteln (48) für die Erzeugung eines zehnten Signals, welches der Summe der achten und neunten Signale proportional ist, Mitteln (54) für die Erzeugung eines elften Signals, welches der spezifischen Wärme von Wasserdampf proportional ist, Mitteln (52) für die Erzeugung eines zwölften Signals, welches dem Produkt der zweiten und elften Signale proportional ist, und Mitteln (50) für die Erzeugung eines dreizehnten Signals, welches dem Produkt des zehnten und zwölften Signals und dem Wärmeverlust infolge der Feuchtigkeit im Brennstoff und der für die Verbrennung zugeführten Luft pro Einheitsgewicht Brennstoff proportional ist.

4. Anlage nach Anspruch 3 mit Mitteln (59) für die Erzeugung eines vierzehnten Signals, welches der latenten Verdampfungswärme von Wasser im Brennstoff proportional ist, und Mitteln (58) für die Erzeugung eines fünfzehnten Signals, welches dem Produkt des neunten und vierzehnten Signals proportional ist sowie dem Verlust infolge der latenten Wärme der Verdampfung des Wassers im Brennstoff.

5. Anlage nach Anspruch 4 mit Mitteln (72) für die Erzeugung eines sechzehnten Signals, welches der Geschwindigkeit der Dampfströmung aus dem Generator proportional ist, und mit einem Funktionsgenerator (74), welcher auf das sechszehnte Signal anspricht für die Erzeugung eines siebzehnten Signals, welches sich in umgekehrtem, nicht linearem Verhältnis zur Größe des sechzehnten Signals verändert sowie zum Wärmeverlust in Prozent infolge der Wärmestrahlung aus dem Dampfgenerator.

6. Anlage nach Anspruch 5 mit Mitteln (70) zum Erzeugen eines achtzehnten Signals, welches dem Wärmeverlust proportional ist, der von unverbranntem Kohlenstoff in Asche stammt, die sich aus der Verbrennung des Brennstoffes ergibt.

7. Anlage nach Anspruch 6 mit Mitteln (24, 60) für die Erzeugung eines neunzehnten Signals, welches proportional ist der Summe des vierten, siebenten, dreizehnten, fünfzehnten und achtzehnten Signals, Mitteln (82) für die Erzeugung eines zwanzigsten Signals, welches proportional ist dem höheren Heizwert von Brennstoff, Mitteln (81) für die Erzeugung eines einungzwanzigsten Signals, welches proportional ist zum neunzehnten Signal, dividiert durch das zwanzigste Signal, Mitteln (80) für die Erzeugung eines zweiundzwanzigsten Signals, welches proportional ist der Summe des siebzehnten und einundzwanzigsten Signals sowie der Summe der Wärmeverluste in Prozent, Mitteln (84) für die Erzeugung eines dreiundzwanzigsten Signals, welches proportional dem 100% Wirkungsgrad des Dampfgenerators ist, und Mitteln (36) für die Erzeugung eines vierzehnten Signals, welches proportional ist zur Differenz zwischen dem dreiundzwanzigsten und zweiundzwanzigsten Signal und das Signal bildet, welches dem Wirkungsgrad des Dampfgenerators proportional ist.

## Revendications

1. Système pour la détermination automatique et en continu du rendement d'une chaudière à vapeur chauffée au combustible fossile, le système agissant pour générer une série de signaux de perte de chaleur proportionnels à une série de pertes de chaleur différentes et pour additionner les signaux de perte de chaleur afin de fournir un signal proportionnel au rendement de la chaudière à vapeur, et le système comportant des moyens (25) pour générer un premier signal proportionnel aux unités de poids de gaz brûlés de sortie secs par unité de poids de combustible, des moyens (12, 14, 16) pour générer un second signal correspondant à la différence de température entre les gaz brûlés de sortie et l'air fourni pour la combustion, des moyens (20) pour générer un troisième signal proportionnel à la chaleur spécifique des gaz brûlés de sortie secs, et des moyens (18, 22) pour générer un quatrième signal proportionnel au produit des premier, second et troisième signaux et à la perte de chaleur apparue dans les gaz brûlés secs en unités de chaleur par poids unitaire de combustible.

2. Système selon la revendication 1, caractérisé en ce qu'il comporte de moyens (62, 64) pour générer un cinquième signal proportionnel aux unités de poids de corps combustibles présents dans les gaz brûlés de sortie par poids unitaire de combustible, des moyens (68) pour générer un sixième signal proportionnel à la chaleur de combustion des corps combustibles, et des moyens (66) pour générer un septième signal proportionnel au produit des cinquième et sixième signaux et à la perte de chaleur due aux corps combustibles présents dans les gaz brûlés de sortie en unités de chaleur par poids unitaire de combustible.

3. Système selon la revendication 2, caractérisé en ce qu'il comporte des moyens (26, 30) pour générer un huitième signal proportionnel aux unités de poids d'humidité présente dans l'air fourni pour la combustion par poids unitaire de combustible, des moyens (47) pour générer un neuvième signal proportionnel aux unités de poids d'humidité présente dans le combustible par poids unitaire de combustible, des moyens (48) pour générer un dixième signal proportionnel à la somme des huitième et neuvième signaux, des moyens (54) pour générer un onzième signal proportionnel à la chaleur spécifique de la vapeur d'eau, des moyens (52) pour générer un douzième signal proportionnel au produit des second et onzième signaux, et des moyens (50) pour générer un treizième signal proportionnel au produit des dizième et douzième signaux et à la perte de chaleur due à l'humidité présente dans le combustible et dans l'air fourni pour la combustion par poids unitaire de combustible.

4. Système selon la revendication 3, caractérisé en ce qu'il comporte des moyens (59) pour générer un quatorzième signal proportionnel à la chaleur latente de vaporisation de l'eau présente dans le combustible, et des moyens (58) pour générer un quinzième signal proportionnel au produit des neuvième et quatorzième signaux et à la perte due à la chaleur latente de vaporisation de l'eau présente dans le combustible.

5. Système selon la revendication 4, caractérisé en ce qu'il comporte des moyens (72) pour générer un seizième signal proportionnel au débit d'écoulement de vapeur à partir de la chaudière, et un générateur de fonction (74) sensible au seizième signal pour générer un dix-septième signal variant en fonction inverse non linéaire de l'amplitude du seizième signal et de la perte de chaleur en pourcentage due au rayonnement de chaleur à partir de la chaudière à vapeur.

6. Système selon la revendication 5, caractérisé en ce qu'il comporte des moyens (70) pour générer un dix-huitième signal proportionnel à la perte de chaleur due au carbone non brûlé présent dans les cendres résultant de la combustion du combustible.

7. Système selon la revendication 6, caractérisé en ce qu'il comporte des moyens (24, 60) pour générer un dix-neuvième signal proportionnel à la somme des quatrième, septième, treizième, quinzième et dix-huitième signaux, des moyens (82) pour générer un vingtième signal proportionnel au pouvoir calorifique supérieur du combustible, des moyens (81) pour générer un vingt-et-unième signal proportionnel au dix-neuvième signal divisé par le vingtième signal, des moyens (80) pour générer un vingt-deuxième signal proportionnel à la somme du dix-septième et du vingt-et-unième signaux, et au total desdites pertes de chaleur en pourcentage, des moyens (84) pour générer un vingt-troisième signal proportionnel à un rendement de 100% de la chaudière à vapeur, et des moyens (86) pour générer un vingt-quatrième signal qui est proportionnel à la différence entre les vingt-troisième et vingt-deuxième signaux et constitue le dit signal proportionnel au rendement de la chaudière à vapeur.